# EUROPEAN PATENT APPLICATION

(11) **EP 4 721 661 A1**
(43) Date of publication of application: **08.04.2026**
(21) Application number: 24810962.1
(22) Date of filing: 14.05.2024
(51) Int. Cl.: A61B 5/151

(54) **BLOOD COLLECTION DEVICE AND BLOOD COLLECTION METHOD**

(30) Priority: 24.05.2023 JP 2023085449
(71) Applicant: HITACHI HIGH-TECH CORPORATION, Tokyo 105-6409 (JP); Fujita Academy, Toyoake-shi, Aichi 470-1192 (JP)
(72) Inventor: TAKENAKA, Kei, Tokyo 100-8280 (JP); OYAMADA, Yuji, Tokyo 100-8280 (JP); HOKARI, Junpei, Tokyo 100-8280 (JP); YANAGIDA, Atsushi, Hitachi-shi, Ibaraki 317-0077 (JP); ITO, Hiroyasu, Toyoake-shi, Aichi 470-1192 (JP)
(74) Representative: MERH-IP Matias Erny Reichl Hoffmann Patentanwälte PartG mbB
(86) International application number: PCT/JP2024/017715
(87) International publication number: WO 2024/241955

(57) **Abstract**

Provided are a blood collection device and a blood collection method enabling blood collection from any subject in the necessary amount for a test. The blood collection device is provided with a compression mechanism that independently compresses the base section and/or the middle section and the distal section of a finger, and collects blood from a puncture mark on the fingertip by repeatedly compressing the finger in the order of the base section and/or the middle section and the distal section. This blood collection device is characterized in that a period of time for compressing the distal section is longer than a period of time for compressing the middle section and/or a period of time for compressing the base section.

## Description

### Technical Field

The present invention relates to a blood collection device and a blood collection method for collecting blood from a finger of a subject.

### Background Art

To live a healthy life, it is important to regularly have a health checkup. One of tests generally performed during health checkups is a blood test for diagnosing conditions of tissues and organs throughout an entire body by collecting blood from a subject and analyzing components of the blood. General blood tests are often performed at medical institutions, but self-examination, in which measurement items are limited to measurement items such as a blood glucose level, is often performed at home.

A blood collection method that is often performed as blood collection for self-examination, such as measurement of a blood glucose level, is capillary blood collection. The capillary blood collection is a method in which a dedicated skin puncture device is pressed against a finger of a subject to puncture a capillary in the finger and collect blood that has flowed out. The capillary blood collection is a simpler method of collecting blood than venous blood collection. However, the amount of blood that can be ensured is small. Therefore, to ensure the amount of blood, it is necessary to perform a cumbersome operation such as compressing and squeezing the finger in order to collect blood.

Patent Literature 1 discloses a blood collection device that automates blood collection from a finger. This blood collection device includes a cartridge that holds a puncture device, a blood collection tube, and an adhesive bandage, a drive mechanism that changes the position of the cartridge and presses the puncture device, a container, and a sticker against a fingertip, a fixing mechanism for fixing a portion of the fingertip, and a compression mechanism for compressing the base of the finger. After compressing the fingertip, the fingertip of the subject is punctured with the puncture device, blood is collected from a puncture mark into the blood collection tube, and after release from the compression, bleeding at the puncture mark is stopped with the adhesive bandage. In the blood collection, by repeatedly compressing and releasing the distal section of the finger and changing the relative distance between the puncture mark on the fingertip and the blood collection tube at regular time intervals, droplets of blood flowing out from the puncture mark can be efficiently collected into the blood collection tube, and a blood coagulation reaction at the puncture mark can be suppressed, and therefore the amount of blood collected can be increased.

In addition, Patent Literature 2 discloses a blood collection device that compresses a finger with a plurality of tightening members, and transfers the compression in order from the tightening member farthest from a puncture site to the tightening member closest to the puncture site, thereby drawing blood from the puncture site.

### Citation List

### Patent Literature

Patent Literature 1: Japanese Unexamined Patent Application Publication No. 2019-088391
Patent Literature 2: Japanese Unexamined Patent Application Publication No. 2005-278740

### Summary of Invention

### Technical Problem

One of functions required for a blood collection device is to ensure the amount of blood required for a blood test in one blood collection. To address this issue, the inventors have conducted studies on an effective method for compressing a finger.

For the blood collection device described in Patent Literature 1 described above, compression of multiple sites and control of a period of time for compression are not described. Meanwhile, for the blood collection device described in Patent Literature 2, a compression mechanism for a distal section of a finger, where capillaries are most densely concentrated, is not provided, and a period of time for compressing each section using multiple tightening members is not described.

Further, the above-described Patent Literature 1 and Patent Literature 2 do not describe a structure of a compression unit that is effective for an increase in the amount of blood collected, and do not describe a mechanism that controls compression of a finger based on bleeding from the finger and a condition of a blood vessel in the fingertip.

Therefore, the present invention provides a blood collection device and a blood collection method that can ensure the amount of collected blood required for a test from any subject.

### Solution to Problem

To solve the above-described problems, a blood collection device according to the present invention that collects blood from a puncture mark on a fingertip by repeatedly compressing a finger in order of a base section and/or a middle section and a distal section of the finger includes a compression mechanism that independently compresses the base section and/or the middle section and the distal section of the finger, in which a period of time for compressing the distal section is longer than a period of time for compressing the middle section and/or a period of time for compressing the base section.

In addition, a blood collection method according to the present invention collects blood from a puncture mark on a fingertip by causing a compression mechanism configured to independently compress a base section and/or a middle section and a distal section of a finger to repeatedly compress the finger in order of the base section and/or the middle section and the distal section, and a period of time for compressing the distal section is longer than a period of time for compressing the middle section and/or a period of time for compressing the base section.

### Advantageous Effects of Invention

According to the present invention, it is possible to provide a blood collection device and a blood collection method that can ensure the amount of collected blood required for a test from any subject.

Objects, configurations, and effects other than the above will be apparent from the description of the following embodiments.

### Brief Description of Drawings

Fig. 1 is a diagram illustrating an exterior of a blood collection device according to a first embodiment of the present invention.
Fig. 2 is a diagram illustrating a configuration of a turntable according to the first embodiment of the present invention.
Fig. 3 is a schematic configuration diagram of the blood collection device according to the first embodiment of the present invention.
Fig. 4 is a diagram illustrating periods of time for compressing a base section, a middle section, and a distal section according to the first embodiment of the present invention.
Fig. 5 is a diagram illustrating a procedure for compressing the distal section based on a finger blood vessel image according to the first embodiment of the present invention.
Fig. 6 is a diagram illustrating a procedure for compressing the distal section based on a blood image according to the first embodiment of the present invention.
Fig. 7 is a cross-sectional view of a distal section compression mechanism included in the blood collection device according to the first embodiment of the present invention.
Fig. 8 is a cross-sectional view of the distal section compression mechanism included in the blood collection device according to the first embodiment of the present invention.
Fig. 9 is a cross-sectional view of the distal section compression mechanism included in the blood collection device according to the first embodiment of the present invention.
Fig. 10 is a flowchart of a blood collection operation checking process by the blood collection device according to the first embodiment of the present invention.
Fig. 11 illustrates a finger setting process, a puncture process, a blood collection process, and a hemostasis process in a flowchart of blood collection by the blood collection device according to the first embodiment of the present invention.
Fig. 12 is a flowchart of the blood collection by the blood collection device according to the first embodiment of the present invention.
Fig. 13 is a flowchart of the blood collection by the blood collection device according to the first embodiment of the present invention.
Fig. 14 is a flowchart of the blood collection by the blood collection device according to the first embodiment of the present invention.

### Description of Embodiments

A blood test that can diagnose conditions of tissues and organs of an entire body is important for maintaining a healthy life. Since blood collection from a subject is essential for conducting a blood test, it is preferable that anyone can collect blood easily and safely. Therefore, the inventors of the present invention have conducted various studies on a method and a device for automatic blood collection and have arrived at the present invention. Preferred embodiments thereof will be described below.

The embodiments of the present invention will be described below with reference to the drawings. The embodiments described below are examples, and it goes without saying that other aspects are possible by combining or substituting with publicly known or well-known techniques.

### First Embodiment

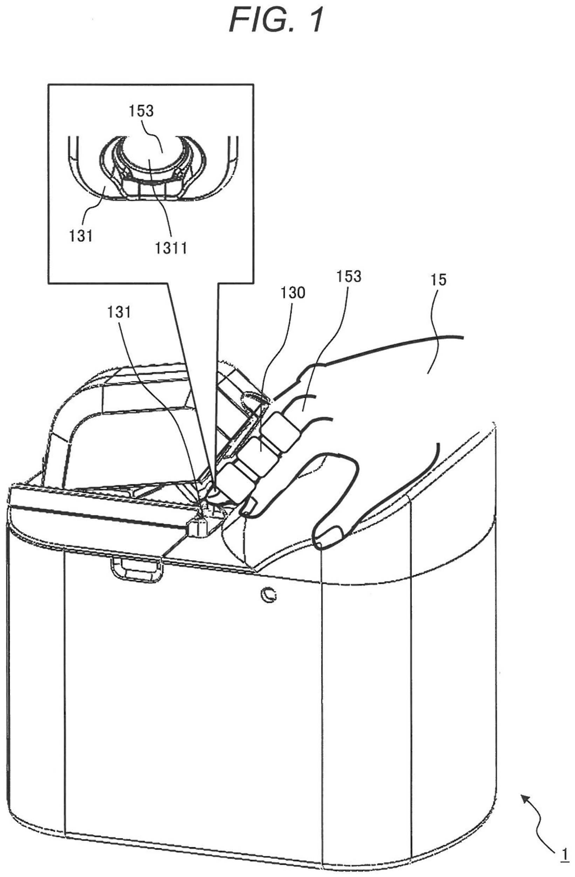

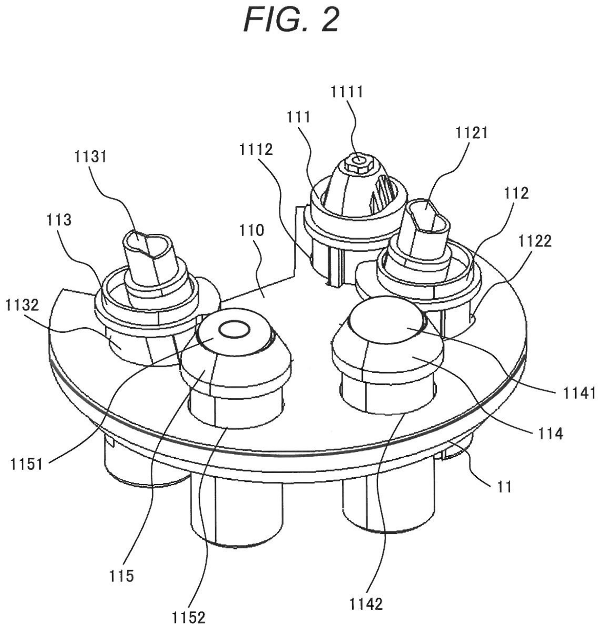

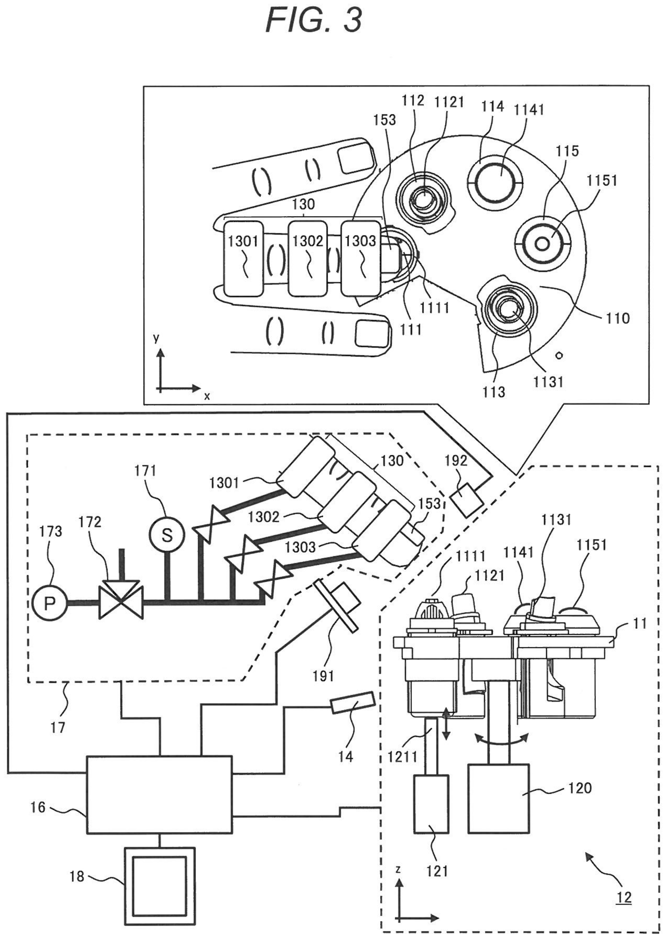

First, a basic configuration of a blood collection device 1 according to the present invention will be described. Fig. 1 is an exterior diagram of the blood collection device 1 during collection of blood from a finger. The blood collection device 1 includes a turntable 11 including a puncture device (Fig. 2), a blood collection tube (Fig. 2), gauze (Fig. 2), and the like, a drive mechanism 12 (Fig. 3) that controls a movement of the turntable 11, a finger compression mechanism 130 that tightens a periphery of a finger 153, and a pressure adjustment mechanism 17 (Fig. 3) for adjusting pressure of the finger compression mechanism 130 (Fig. 3).

For collection of blood from the finger, a finger rest 131 that is a disposal component is set on the blood collection device 1, then the finger 153 of a hand 15 is placed on the finger rest 131, and the blood collection device 1 is used. A speech bubble diagram in Fig. 1 is a diagram when the finger rest 131 is viewed from below. The finger rest 131 includes a blood collection window 1311 that is a void for blood collection. The blood collection device 1 punctures the finger 153 and collects blood from the finger 153 by bringing the puncture device (Fig. 2) and the blood collection tube (Fig. 2) into contact with the finger 153 via the blood collection window 1311.

Next, a configuration of the turntable 11 used by the blood collection device 1 according to the present embodiment will be described with reference to Fig. 2 that is an assembly diagram of the turntable 11.

The turntable 11 holds a puncture device holder 111, a complete blood count blood collection tube holder 112, a biochemistry/immunology blood collection tube holder 113, a gauze holder 114, and an adhesive bandage holder 115 in a puncture device holder holding hole 1112, a complete blood count blood collection tube holder holding hole 1122, a biochemistry/immunology blood collection tube holder holding hole 1132, a gauze holder holding hole 1142, and an adhesive bandage holder holding hole 1152 of the turntable 11. The puncture device holder 111 holds the puncture device 1111. The complete blood count blood collection tube holder 112 holds a complete blood count blood collection tube 1121. The biochemistry/immunology blood collection tube holder 113 holds a biochemistry/immunology blood collection tube 1131 that holds the biochemistry/immunology blood collection tube 1131. The gauze holder 114 holds gauze 1141, the adhesive bandage holder 115 holds an adhesive bandage 1151 (also referred to as a sticker). The center of each of the holding holes is on the circumference of a circle whose center is the rotation axis of the turntable 11. Each of the holding holes is a through-hole. However, since portions of the puncture device holder 111, the complete blood count blood collection tube holder 112, the biochemistry/immunology blood collection tube holder 113, the gauze holder 114, and the adhesive bandage holder 115 are larger than the respective holding holes, the puncture device holder 111, the complete blood count blood collection tube holder 112, the biochemistry/immunology blood collection tube holder 113, the gauze holder 114, and the adhesive bandage holder 115 do not fall from the respective holding holes.

The puncture device 1111 is a single-use skin puncture device including a puncture needle and a holder housing the puncture needle. When a tip of the puncture device 1111 is pressed against the finger, the puncture needle pops out for a moment and punctures the skin and capillaries of the finger. The complete blood count blood collection tube 1121 and the biochemistry/immunology blood collection tube 1131 are containers for blood collection, and collect blood from a puncture site by pressing openings of the blood collection tubes against the puncture site on the finger. The complete blood count blood collection tube 1121 is a blood collection tube with an inner surface coated with an anticoagulant and has an effect of suppressing a blood coagulation reaction after blood collection. The biochemistry/immunology blood collection tube 1131 is a blood collection tube housing a separating agent and can separate blood cells from serum due to a difference in specific gravity by applying centrifugal force after blood collection. The gauze 1141 is an absorbent cloth. By pressing the gauze 1141 against the finger 153, excess blood is wiped off from the puncture site on the finger 153. The adhesive bandage 1151 has an absorbent pad at the center of an adhesive sheet, and covers the puncture site on the finger 153. The adhesive bandage 1151 is set in the adhesive bandage holder 115 such that an adhesive surface faces upward.

In addition, to prevent blood from adhering to the turntable 11 during blood collection, a disposable protective sheet 110 may be placed on the turntable 11, and the turntable 11 may be used. The protective sheet 110 has through-holes at positions corresponding to the respective holding holes of the turntable 11 and is made of an inexpensive and light material such as paper, cloth, or a resin film.

Fig. 3 is a schematic configuration diagram of the blood collection device 1 according to the present embodiment and illustrates an internal configuration. As illustrated in Fig. 3, the blood collection device 1 (Fig. 1) includes the turntable 11, the drive mechanism 12, the finger compression mechanism 130, the pressure adjustment mechanism 17, a collected blood amount measurement mechanism 14, a finger blood vessel capture mechanism (not illustrated), a control mechanism 16, and an input and output device 18. The turntable 11 includes the puncture device 1111, the complete blood count blood collection tube 1121, the biochemistry/immunology blood collection tube 1131, the gauze 1141, and the adhesive bandage 1151. The drive mechanism 12 controls a movement of the turntable 11. The finger compression mechanism 130 tightens the periphery of the finger 153 (Fig. 1). The pressure adjustment mechanism 17 adjusts pressure of the finger compression mechanism 130. The collected blood amount measurement mechanism 14 optically measures an amount of blood collected into the complete blood count blood collection tube 1121, the biochemistry/immunology blood collection tube 1131. The finger blood vessel capture mechanism is a mechanism for capturing a blood vessel image of the fingertip of the finger 153. The control mechanism 16 has a function of analyzing the captured image and controlling operations of the drive mechanism 12 and the pressure adjustment mechanism 17. The input and output device 18 displays the captured image, an entry of an instruction into the control mechanism 16, and an output result from the control mechanism 16. As the input and output device 18, a personal computer or a tablet PC connected to the blood collection device 1 can be used.

The drive mechanism 12 includes a rotation direction drive mechanism 120 and a vertical direction drive mechanism 121.

The rotation direction drive mechanism 120 rotates (around the z axis) and moves the turntable 11 such that the puncture device 1111, the complete blood count blood collection tube 1121, the biochemistry/immunology blood collection tube 1131, the gauze 1141, or the adhesive bandage 1151 is disposed immediately below the blood collection window 1311 (Fig. 1) of the finger rest 131 (Fig. 1) on which the finger 153 targeted for blood collection is to be placed. The vertical direction drive mechanism 121 includes a push rod 1211 and moves, in the vertical direction (z direction) and via the push rod 1211, the puncture device 1111, the complete blood count blood collection tube 1121, the biochemistry/immunology blood collection tube 1131, the gauze 1141, or the adhesive bandage 1151 disposed immediately below the blood collection window 1311 (Fig. 1), and brings it close to the finger 153 or presses it against the finger 153. The puncture device 1111 is pressed against the finger 153 to puncture the finger 153, and the complete blood count blood collection tube 1121 or the biochemistry/immunology blood collection tube 1131 is brought close to the finger 153 to collect blood from the puncture site without causing blood from scattering, the gauze 1141 is pressed against to the finger 153 to wipe off excess blood, and the adhesive bandage 1151 is pressed against the finger 153 to stop bleeding from the puncture site. A power source of the drive mechanism 12 may be an electrical energy source such as an external power source or an internal battery, or may be a mechanical power source such as a spring. In a case where the spring is used as the power source, the power source can be used in a region where it is difficult to supply electricity.

The pressure adjustment mechanism 17 includes the finger compression mechanism 130, a compression cuff pressure sensor 171 for measuring internal pressure of each compression cuff constituting the finger compression mechanism 130, and a compression cuff valve 172 and a compression cuff pump 173 that are configured to control the internal pressure of each compression cuff. By controlling the internal pressure of each compression cuff, it is possible to adjust pressure for tightening the finger 153 targeted for blood collection. In addition, the finger compression mechanism 130 includes a base section compression cuff 1301 that compresses the base section of the finger 153, a middle section compression cuff 1302 that compresses the middle section of the finger 153, and a distal section compression cuff 1303 that compresses the distal section of the finger 153. Each of the compression cuffs can independently compress and release a respective one of the sections by adjusting opening and closing of a valve coupled to the compression cuff.

The collected blood amount measurement mechanism 14 is a device that measures an image and light absorbance of blood in the blood collection tubes.

In the present embodiment, a collected blood amount measurement camera is used and the control mechanism 16 analyzes the image captured by the collected blood amount measurement camera to derive the amount of blood collected.

The finger blood vessel capture mechanism includes a finger blood vessel image capture camera 191, which is an infrared camera, and a finger blood vessel image capture light source 192, which is a near-infrared light source. The finger blood vessel image capture camera 191 and the finger blood vessel image capture light source 192 are disposed opposite to each other with the finger 153 therebetween.

Since hemoglobin in a blood vessel of the finger has the ability to absorb near-infrared light, the blood vessel is displayed as a dark line in the image of the finger 153 captured by the finger blood vessel image capture camera 191. By analyzing the image captured by the finger blood vessel image capture camera 191 with the control mechanism 16, the position of the blood vessel of the finger can be identified, the puncture device 1111 can be moved to the position, and the finger can be punctured.

In addition, the blood collection device 1 has a function of recording a result of the analysis by the collected blood amount measurement mechanism 14 and the operation of the drive mechanism 12 and thereby measuring amounts of blood collected in the complete blood count blood collection tube 1121 and the biochemistry/immunology blood collection tube 1131, and can change the operation based on information indicating the position of the fingertip placed and the amount of blood collected.

Next, a method of compressing the finger 153 in order to obtain a sufficient amount of blood collected by using the blood collection device 1 will be described. Since capillaries are densely concentrated in the distal section of the finger, it is important to efficiently compress this site in order to ensure the amount of blood collected. However, even when the amount of blood included in the capillaries in the distal section of the finger decreases, and the distal section of the finger is continuously compressed, the amount of blood from the puncture site on the finger 153 decreases. Therefore, it is necessary to release the compression of the distal section of the finger and send blood into the capillaries in the distal section of the finger. By repeating the compression and release of the distal section of the finger in the above-described manner, it is possible to continuously collect blood.

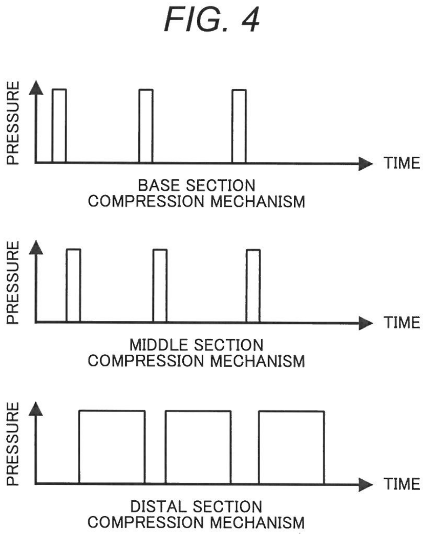

However, in this method, it is necessary to release the finger 153 until blood is sufficiently sent into the capillaries in the finger, and it is expected that the blood coagulation reaction at the puncture site may progress during that time, and that the amount of blood lost may decrease. Therefore, the inventors thought that a period of time for the release could be shortened by actively sending blood into the capillaries in the distal section of the finger at the time of the release of the distal section. The method devised will be described with reference to Fig. 4.

Fig. 4 is a diagram illustrating periods of time for compression by the base section compression cuff 1301, the middle section compression cuff 1302, and the distal section compression cuff 1303 that constitute the finger compression mechanism 130. As illustrated in Fig. 4, the finger is compressed in the order of the base section, the middle section, and the distal section, and the period of time for compressing the distal section is longer than the periods of time for compressing the other sections. By this method, it is possible to actively send blood into the capillaries in the distal section of the finger at the time of the release of the distal section of the finger, and increase the amount of blood collected. From the experimental studies of the inventors, it is preferable that the periods of time for compressing the base section and the middle section be less than 1 second, and that the period of time for compressing the distal section be greater than or equal to 1 second.

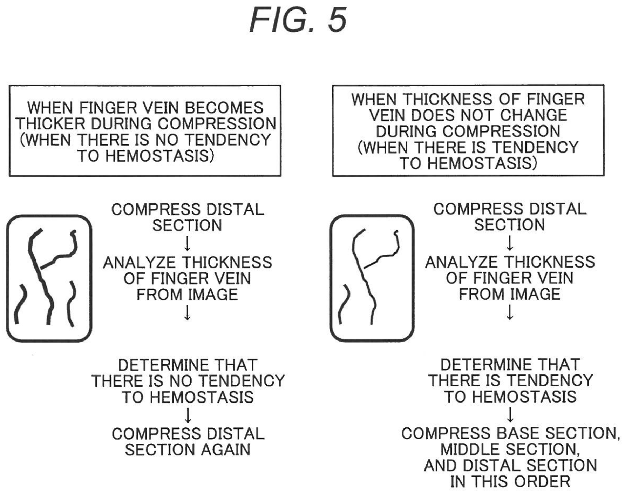

To increase the amount of blood collected, it is preferable to adjust the compression method based on the amount of blood included in the distal section of the finger. One of methods for this will be described with reference to Fig. 5. Fig. 5 is a diagram illustrating a procedure for compressing the distal section using information indicating a thickness of the blood vessel of the finger. A left diagram in Fig. 5 illustrates a compression procedure when the blood vessel of the finger becomes thick during the compression. In this case, it is determined, based on a result of analyzing an image of the blood vessel of the finger, that the amount of blood included in the blood vessel of the finger is large and that there is no tendency to hemostasis, and the compression of the distal section is continued. On the other hand, a right diagram in Fig. 5 illustrates a compression procedure when the thickness of the blood vessel of the finger does not change during the compression. In this case, it is determined, based on a result of analyzing an image of the blood vessel of the finger, that the amount of blood included in the blood vessel of the finger is small and that there is a tendency to hemostasis, and the finger is compressed in the order of the base section, the middle section, and the distal section. By the above-described procedure, it is possible to suppress hemostasis at the puncture site and obtain a large amount of blood collected.

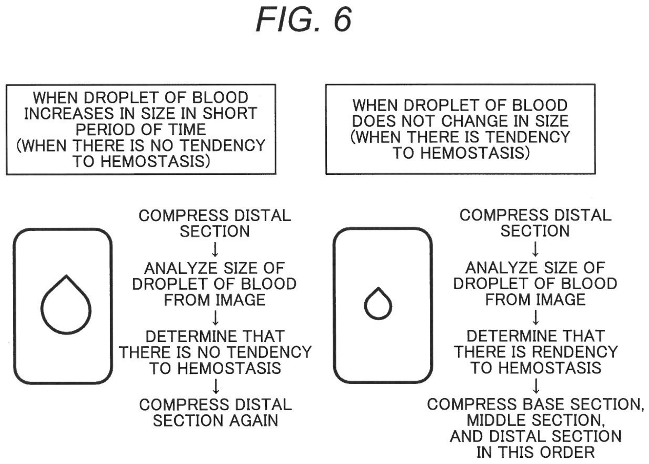

Fig. 6 is a diagram illustrating a procedure for compressing the distal section using information indicating a change in the size of a droplet of blood from the puncture site over time, instead of the image of the blood vessel of the finger. A left diagram in Fig. 6 illustrates a compression procedure when the droplet of blood from the puncture site increases in size in a short period of time. In this case, it is determined, based on a result of analyzing an image of the droplet of blood, that there is no tendency to hemostasis, and the compression of the distal section is continued. On the other hand, a right diagram in Fig. 6 illustrates a compression procedure when the droplet of blood from the puncture site does not change in size. In this case, it is determined, based on a result of analyzing an image of the droplet of blood, that there is a tendency to hemostasis, and the finger is compressed in the order of the base section, the middle section, and the distal section. By the above-described procedure, it is possible to suppress hemostasis and obtain a large amount of blood collected.

In Figs. 5 and 6, the period of time for compressing the distal section is adjusted by changing the thickness of the blood vessel of the finger and the size of a droplet of blood, but the period of time for compressing each of the sections and the strength of the compression may be adjusted.

Next, a compression method suitable for a cross-sectional structure of the finger 153 will be described. The dorsal side of the finger 153 has a hard structure since there are bones and muscles. On the other hand, the palmar side of the finger has many capillaries and a fat layer, and has a soft structure with the capillaries densely concentrated. Therefore, in order to increase the amount of blood collected in the collection of blood from the finger, it is effective to compress the palmar side of the finger where the capillaries are densely concentrated in order to apply pressure to the capillaries. However, when the palmar side of the finger 153 is locally compressed, flesh on the palmar side of the finger 153 spreads to side surfaces of the finger 153 due to the soft structure of the finger 153 on the palmar side, and thus it is not possible to effectively apply pressure to the capillaries in the finger 153. Therefore, it is considered important to compress the palmar side of the finger 153 while pressing the side surfaces of the finger 153 to prevent the flesh of the finger 153 from spreading.

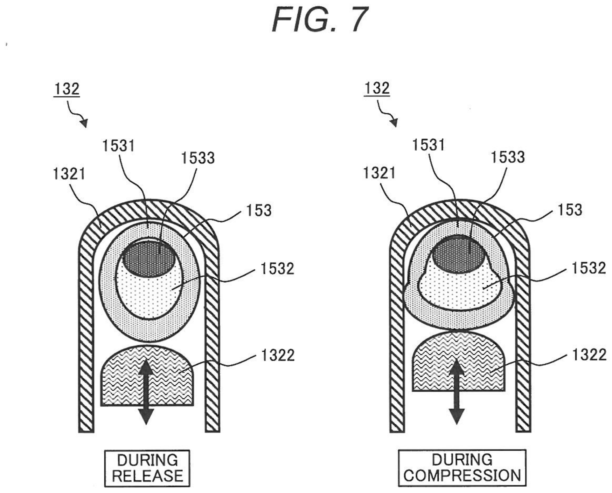

Fig. 7 is a cross-sectional view of a compression component 132 for the finger's distal section devised based on the above-described assumption. The compression component 132 includes a finger dorsal surface fixing unit 1321, which is a shell-like structure, and a finger pad compression unit 1322 movable upward and downward. The finger 153 is placed between the finger dorsal surface fixing unit 1321 and the finger pad compression unit 1322. The surface of the finger 153 is covered with subcutaneous tissue 1531, fat 1532 is present on the palmar side of the inside of the subcutaneous tissue 1531, and a bone 1533 is present on the dorsal side. The capillaries are densely concentrated in the subcutaneous tissue 1531 on the palmar side of the finger 153. In the present embodiment, the finger pad compression unit 1322 is configured to move upward and downward, but is not limited thereto. The finger pad compression unit 1322 may be fixed and the finger dorsal surface fixing unit 1321 may be configured to move upward and downward. That is, the finger dorsal surface fixing unit 1321 and the finger pad compression unit 1322 may be configured such that a relative distance between the finger dorsal surface fixing unit 1321 and the finger pad compression unit 1322 can be changed.

The finger 153 is fixed on an inner surface of the finger dorsal surface fixing unit 1321, and the palmar side of the finger 153 is compressed by moving the finger pad compression unit 1322 upward. Since the finger dorsal surface fixing unit 1321 presses the side surfaces of the finger 153, the flesh of the pad of the finger 153 does not spread to the side surfaces of the finger 153, and the palmar side of the finger 153 can be effectively compressed.

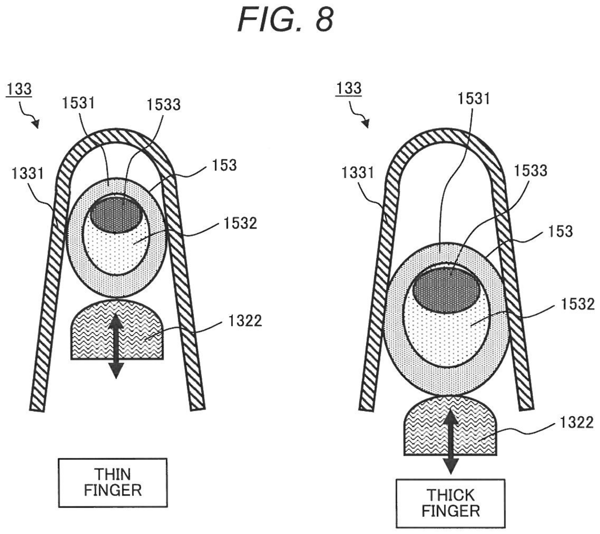

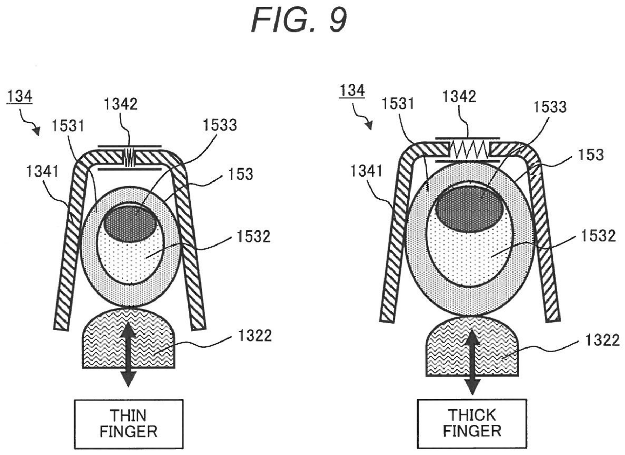

Next, Figs. 8 and 9 illustrate structures of compression components that can compress the palmar side of the finger regardless of the thickness of the finger 153 of the subject.

Fig. 8 is a cross-sectional view of a tapered compression component 133. A tapered finger dorsal surface fixing unit 1331 of the tapered compression component 133 has a tapered structure. By pressing the finger 153 against an inner surface of the tapered finger dorsal surface fixing unit 1331, the finger dorsal surface fixing unit 1331 can press the side surfaces of the finger 153 regardless of the thickness of the finger 153. Therefore, by moving the finger pad compression unit 1322 upward, the flesh of the pad of the finger 153 does not spread to the side surfaces of the finger 153, and the palmar side of the finger 153 can be efficiently compressed. In other words, the tapered finger dorsal surface fixing unit 1331 has a shape in which a distance between surfaces that come into contact with both side surfaces of the finger 153 becomes shorter toward the dorsal side of the finger 153.

Fig. 9 is a cross-sectional view of an adjustable compression component 134. An adjustable finger dorsal surface fixing unit 1341 of the adjustable compression component 134 includes, at its middle portion, a stretchable unit 1342 that is stretchable. The stretchable unit 1342 is equipped with a spring inside thereof and changes its length based on the thickness of the set finger 153 when the spring stretches. Therefore, regardless of the thickness of the finger 153, the finger dorsal surface fixing unit 1331 can press the side surfaces of the finger 153, and the palmar side of the finger 153 can be efficiently compressed by moving the finger pad compression unit 1322 upward, while the flesh of the pad of the finger 153 does not spread to the side surfaces of the finger 153. In other words, the adjustable finger dorsal surface fixing unit 1341 that comes into contact with both surfaces of the finger 153 has a structure in which a distance between surfaces that come into contact with both side surfaces of the finger 153 is adjustable.

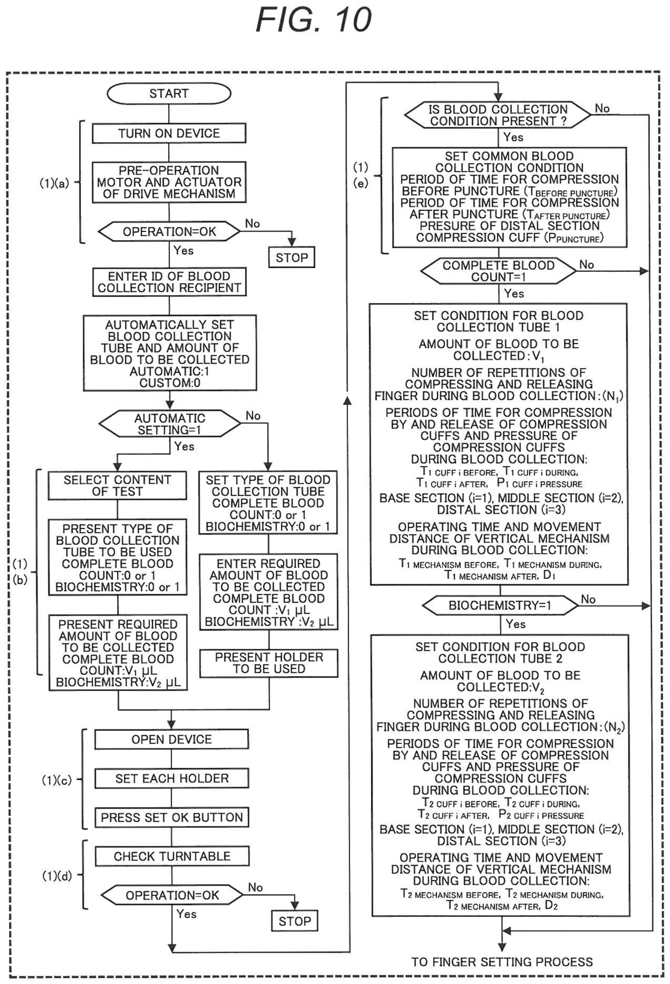

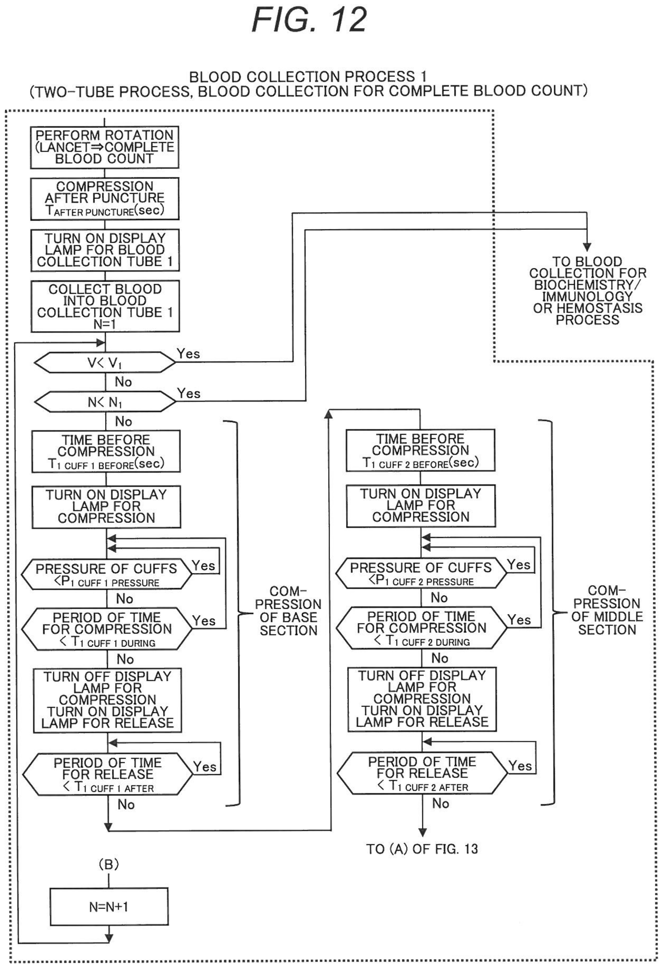

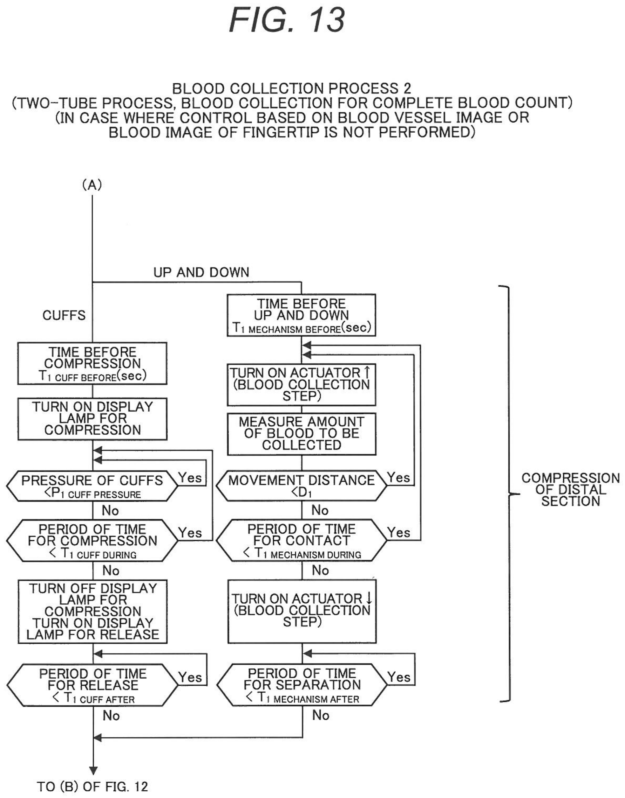

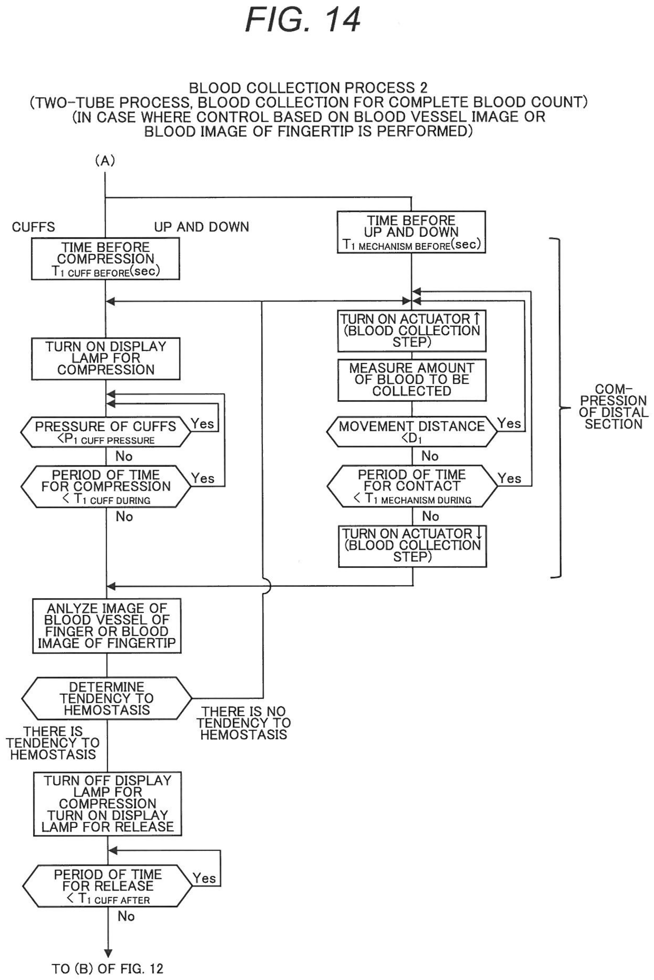

Figs. 10 to 14 are flowcharts of a blood collection step of collecting blood from the finger using the blood collection device 1. In the flowcharts, the following numbers corresponding to respective steps are described. The blood collection by the blood collection device 1 is performed in the order of (1) an operation checking process, (2) a finger setting process, (3) a puncture process, (4) a blood collection process, and (5) a hemostasis process. The blood collection device 1 can be operated by the subject or a person other than the subject. The blood collection step of the blood collection device 1 will be described with reference to the flowcharts of Figs. 10 to 14.
(1) Operation Checking Process (Fig. 10)
   (a) First, when the device is turned on, the rotation direction drive mechanism 120 (Fig. 3) and the vertical direction drive mechanism 121 (Fig. 3) operate and check operations of the respective drive mechanisms. In a case where an abnormality is present in the operation checking, the operations are stopped.
   (b) Next, the user selects the content of a test to be conducted. Based on the content of the test selected, the blood collection device 1 shows the type of a blood collection tube to be used and the amount of blood to be collected. The content of a test may not be selected and the type of a blood collection tube to be used and the amount of blood to be collected may be customized and set.
   (c) On the turntable 11, each of the holders such as the puncture device holder 111 holding the puncture device 1111 is set. After the setting, the process proceeds to checking of the turntable 11.
   (d) The rotation direction drive mechanism 120 (Fig. 3) and the vertical direction drive mechanism 121 (Fig. 3) operate and check whether the puncture device holder 111 (Fig. 2), the complete blood count blood collection tube holder 112 (Fig. 2), the biochemistry/immunology blood collection tube holder 113 (Fig. 2), the gauze holder 114 (Fig. 2), and the adhesive bandage holder 115 (Fig. 2) set on the turntable 11 operate without an abnormality. If an abnormality is not present in the operations, the process proceeds to an entry of an ID of the user.
   (e) The user of the device uses the input and output device 18 to enter the ID of the blood collection recipient. Blood collection conditions vary depending on the subject. If blood collection conditions of the blood collection recipient are not recorded in a memory of the device, or the blood collection conditions are changed, blood collection conditions are entered by using the input and output device 18. In the present embodiment, the blood collection conditions are as follows.

### <Type of Blood Collection Tube>

The complete blood count blood collection tube, the biochemistry/immunology blood collection tube

### <Conditions during Puncture>

Periods of time for compression by the cuffs before and after puncture: T_{before punctures}, T_{after puncture}, and pressure P_{puncture} of the cuffs at this time <Conditions during Blood Collection>

The amount of blood to be collected using the complete blood count blood collection tube: V₁
The number of repetitions of compression by the cuffs and release of the cuffs during blood collection using the complete blood count blood collection tube: N₁
A standby time before compression by the cuffs: T_{1 cuff i before}, periods of time for compression by the cuffs: T_{1 cuff i during}, periods of time for releasing the cuffs: T_{1 cuff i after}, The pressure of the cuffs: P_{1 cuff i pressure} (the base section (i = 1), the middle section (i = 2), and the distal section (i = 3))
The amount of blood collected with the biochemistry/immunology blood collection tube: V₂ The number of repetitions of compression by the cuffs and the release of the cuffs during blood collection with the biochemistry/immunology blood collection tube: N₂ A standby time before compression by the cuffs: T_{2 cuff i before}, periods of time for compression by the cuffs: T_{2 cuff i during}, periods of time for releasing the cuffs: T_{2 cuff i after}, the pressure of the cuffs: P_{2 cuff i pressure} (the base section (i = 1), the middle section (i = 2), and the distal section (i = 3))
After the end of the entry, the process proceeds to the finger setting process.

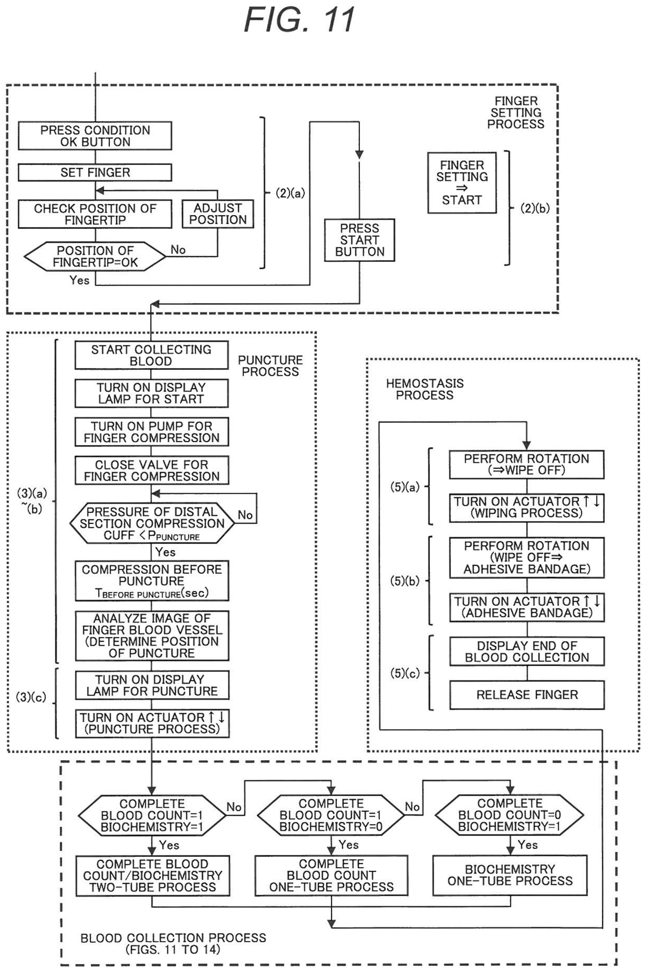

(2) Finger Setting Process (Fig. 11)
(a) In the present embodiment, blood is collected from the middle finger, but the finger targeted for blood collection may be an index finger or a ring finger. First, the fingertip of the finger 153 is placed on the finger rest 131 and whether the fingertip is placed at an appropriate position is checked (Fig. 1).
(b) After the completion of the finger setting process, the operator provides a start instruction via the input and output device 18, and the blood collection device 1 causes the process to proceed to the puncture process.

(3) Puncture Process (Fig. 11)
(a) First, the pressure adjustment mechanism 17 (Fig. 3) increases the inner pressure of the distal section compression cuff 1303 of the finger compression mechanism 130 (Fig. 3) and compresses the distal section of the finger 153 (Fig. 3) targeted for blood collection to collect blood to the fingertip. Since compressing the distal section, where the capillaries are densely concentrated, allows a larger amount of blood to be collected to the fingertip than compressing the base section or the middle section of the finger 153, the distal section is suitable as a range to be compressed.
(b) In parallel with the compression with the distal section compression cuff 1303, the finger blood vessel capture mechanism irradiates the dorsal side of the finger 153 with near-infrared light from the finger blood vessel image capture light source 192 (Fig. 3) and captures an image of the finger 153 using the finger blood vessel image capture camera 191 (Fig. 3). The blood vessel is displayed as a dark line in the image of the finger 153 captured by the finger blood vessel image capture camera 191 (Fig. 3). The control mechanism 16 (Fig. 3) identifies the position of the blood vessel of the finger by analyzing the image captured by the finger blood vessel image capture camera 191 (Fig. 3).
(c) The turntable 11 is rotated to move the puncture device 1111 to a position below the finger 153 (Fig. 3). Thereafter, the position of the puncture device 1111 is finely adjusted to a position immediately below the blood vessel of the finger determined in the previous step. After the position of the puncture device 1111 is finely adjusted, the vertical direction drive mechanism 121 (Fig. 3) pushes the puncture device holder 111 (Fig. 3) toward the fingertip of the finger 153 (Fig. 3) and punctures the fingertip by pressing the puncture device 1111 (Fig. 3) against the finger 153 (Fig. 3).
After the puncture process is completed, the process proceeds to the blood collection process.

(4) Blood Collection Process (Fig. 11, Fig. 12, Fig. 13, and Fig. 14)
(a) Based on blood collection tube conditions entered in the operation checking process, the blood collection process is divided into blood collection processes, which are two-tube blood collection (complete blood count and biochemistry/immunology) and one-tube collection (complete blood count or biochemistry/immunology). In the present embodiment, the case of the two-tube blood collection is described.

The rotation direction drive mechanism 120 (Fig. 3) rotates the turntable 11 (Fig. 3) to move the complete blood count blood collection tube 1121 (Fig. 3) to a position immediately below the finger 153 (Fig. 3). Subsequently, the vertical direction drive mechanism 121 (Fig. 3) pushes the complete blood count blood collection tube holder 112 (Fig. 3) upward toward the fingertip of the finger 153 (Fig. 3) to bring an opening of the complete blood count blood collection tube 1121 (Fig. 3) close to the finger 153 (Fig. 3). When blood from the puncture site on the fingertip comes into contact with the opening of the complete blood count blood collection tube 1121 (Fig. 3), the blood enters into the complete blood count blood collection tube 1121 due to the wettability of the opening of the complete blood count blood collection tube 1121 (Fig. 3).
(b) A method of compressing the finger 153 and a method of operating the blood collection tubes will be described.

As described with reference to Fig. 4, by repeatedly increasing the inner pressure of the base section compression cuff 1301, the middle section compression cuff 1302, and the distal section compression cuff 1303 that constitute the finger compression mechanism 130, the base section, the middle section, and the distal section of the finger 153 are repeatedly compressed in the order of the base section, the middle section, and the distal section. Periods of time for the compression by the base section compression cuff 1301 and the middle section compression cuff 1302 are 0.5 seconds, and a period of time for the compression by the distal section compression cuff 1303 is 3 seconds. The internal pressure of each of the compression cuffs is differential pressure from atmospheric pressure, and is 90 kPa at the time of pressurization and 0 kPa at the time of release.

Although the amount of blood from the fingertip is small immediately after the compression of the base section, blood is concentrated in the fingertip by compressing the finger in the order of the base section, the middle section, and the distal section, and blood from the puncture site forms a droplet of blood at the fingertip. By continuing the compression by the distal section compression cuff 1303, the droplet of blood further increases in size. Immediately before the end of the compression by the distal section compression cuff 1303, the opening of the complete blood count blood collection tube 1121 (Fig. 3) is brought close to the fingertip to bring the droplet of blood into contact with the complete blood count blood collection tube 1121 (Fig. 3). Due to the contact, a part of the droplet of blood falls onto the bottom of the complete blood count blood collection tube 1121, and a part of blood remains between the fingertip and the opening of the complete blood count blood collection tube 1121. After the contact, the inner pressure of the distal section compression cuff 1303 is set to 0 kPa and the fingertip is released from the compression. The blood vessel of the finger 153 tightened by this operation is opened, and thus blood flows in the fingertip again. Thereafter, the opening of the complete blood count blood collection tube 1121 (Fig. 3) is separated from the fingertip. This operation allows blood collected at the fingertip and the opening of the complete blood count blood collection tube 1121 (Fig. 3) to be separated toward the opening of the complete blood count blood collection tube 1121. The separated blood moves toward the bottom of the complete blood count blood collection tube 1121 (Fig. 3) due to gravity. After the opening of the complete blood count blood collection tube 1121 (Fig. 3) is separated from the fingertip, the finger is compressed again in the order of the base section, the middle section, and the distal section. By adding the compression of the base section and the middle section in addition to the compression of the distal section, it is possible to actively send blood into the capillaries in the distal section of the finger at the time of the release of the distal section of the finger and to increase the amount of blood collected. In this manner, blood is collected into the complete blood count blood collection tube 1121 (Fig. 3) by repeatedly performing the compression and release by the finger compression mechanism 130 and changing the relative distance between the fingertip and the complete blood count blood collection tube 1121 (Fig. 3).

By repeating the compression and the release by the finger compression mechanism 130, it is possible to repeat promotion of bleeding from the fingertip and replenishment of blood to the fingertip. In a case where the distance between the complete blood count blood collection tube 1121 (Fig. 3) and the fingertip is not changed, a blood coagulation reaction progresses in blood collected at the fingertip and the opening of the complete blood count blood collection tube 1121 (Fig. 3), and bleeding from the puncture site stops. However, by changing the distance between the complete blood count blood collection tube 1121 (Fig. 3) and the fingertip and removing blood collected at the fingertip and the opening of the complete blood count blood collection tube 1121 (Fig. 3), it is possible to suppress the blood coagulation reaction at the puncture site. As a result, by repeatedly performing the compression and the release by the finger compression mechanism 130 and changing the distance between the complete blood count blood collection tube 1121 (Fig. 3) and the fingertip, it is possible to increase the amount of blood collected.

In the present embodiment, the base section, the middle section, and the distal section are periodically compressed, but a tendency to hemostasis may be determined and a period of time for compressing each of the sections and the strength of the compression may be changed based on analysis of a blood vessel image of the distal section of the finger and the size of a droplet of blood from the puncture site as described with reference to Fig. 5 and Fig. 6.
(c) The amount of blood collected into the complete blood count blood collection tube 1121 (Fig. 3) is measured by the collected blood amount measurement mechanism 14 as described above. When it is determined that a predetermined amount of blood has been collected, the collection of blood into the complete blood count blood collection tube 1121 (Fig. 3) is ended, and the process proceeds to blood collection into the biochemistry/immunology blood collection tube 1131.
(d) The rotation direction drive mechanism 120 (Fig. 3) rotates the turntable 11 to move the biochemistry/immunology blood collection tube 1131 (Fig. 3) to a position immediately below the finger 153. Blood collection into the biochemistry/immunology blood collection tube 1131 (Fig. 3) is performed in a similar operation to the blood collection into the complete blood count blood collection tube 1121 (Fig. 3).
(e) Since the blood coagulation reaction progresses in blood collected into the complete blood count blood collection tube 1121 (Fig. 3), the complete blood count blood collection tube 1121 (Fig. 3) into which blood has been completely collected is extracted during the blood collection into the biochemistry/immunology blood collection tube 1131 (Fig. 3), and inversion mixing is performed. Due to the inversion mixing, an anticoagulant adhering to the inner surface of the complete blood count blood collection tube 1121 (Fig. 3) is mixed with the blood to suppress the blood coagulation reaction. After the blood collection into the biochemistry/immunology blood collection tube 1131 (Fig. 3) is ended, the process proceeds to the hemostasis process.

(5) Hemostasis Process (Fig. 11)
(a) The rotation direction drive mechanism 120 (Fig. 3) rotates the turntable 11 (Fig. 3) to move the gauze 1141 (Fig. 3) to a position immediately below the finger 153. Subsequently, the vertical direction drive mechanism 121 (Fig. 3) pushes the gauze 1141 (Fig. 3) upward toward the fingertip of the finger 153 (Fig. 3) and brings the gauze 1141 (Fig. 3) into contact with the fingertip of the finger 153 (Fig. 3) to wipe off blood remaining on the fingertip.
(b) Thereafter, the turntable 11 (Fig. 3) is rotated to move the adhesive bandage 1151 (Fig. 3) to a position immediately below the blood collection window 1311. Subsequently, the vertical direction drive mechanism 121 (Fig. 3) pushes the adhesive bandage 1151 (Fig. 3) upward toward the fingertip of the finger 153 and applies the adhesive bandage 1151 (Fig. 3) to the fingertip of the finger 153 (Fig. 3) to cover the puncture site on the fingertip. By wiping off blood remaining on the fingertip with the gauze 1141 (Fig. 3) before the puncture site is covered with the adhesive bandage 1151 (Fig. 3), it is possible to prevent blood from overflowing from the adhesive bandage 1151 (Fig. 3) and to reliably attach the adhesive bandage 1151 (Fig. 3) to the fingertip.
(c) After the attachment of the adhesive bandage 1151 (Fig. 3), the subject removes the finger 153 (Fig. 3) from the finger compression mechanism 130 (Fig. 3). Thereafter, the biochemistry/immunology blood collection tube 1131 is removed in a similar manner to the complete blood count blood collection tube 1121 (Fig. 3).

By the above-described procedure using the blood collection device 1, blood is collected into the complete blood count blood collection tube 1121 (Fig. 3) and the biochemistry/immunology blood collection tube 1131 (Fig. 3). In the present embodiment, blood is collected into the two blood collection tubes (complete blood count blood collection tube and biochemistry/immunology blood collection tube), but only blood collection into the complete blood count blood collection tube 1121 (Fig. 3) or only blood collection into the biochemistry/immunology blood collection tube 1131 (Fig. 3) can be performed. In this case, after blood collection into the complete blood count blood collection tube or the biochemistry/immunology blood collection tube is ended, the process proceeds to the hemostasis process.

In the present embodiment, the finger compression mechanism 130 includes the base section compression cuff 1301, the middle section compression cuff 1302, and the distal section compression cuff 1303, and is configured to repeatedly compress the base section, the middle section, and the distal section of the finger 153 in the order of the base section, the middle section, and the distal section, but is not limited thereto. For example, the finger compression mechanism 130 may include the base section compression cuff 1301 or the middle section compression cuff 1302 and the distal section compression cuff 1303 and may be configured to repeatedly compress the base section or the middle section and the distal section of the finger 153 in the order of the base section or the middle section and the distal section. That is, the finger compression mechanism 130 may include the base section compression cuff 1301 and/or the middle section compression cuff 1302 and the distal section compression cuff 1303 and may be configured to repeatedly compress the base section and/or the middle section and the distal section of the finger 153 in the order of the base section and/or the middle section and the distal section. In this case, a period of time for compressing the distal section is longer than a period of compressing the base section and/or a period of compressing the middle section.

As described above, according to the present embodiment, it is possible to provide a blood collection device and a blood collection method that can ensure the amount of blood required for a test from any subject.

The present invention is not limited to the above-described embodiments and include various modifications. For example, the embodiments described above have been explained in detail to simply explain the present invention, and are not necessarily limited to embodiments including all of the configurations described above.

### Reference Signs List

1: Blood collection device
11: Turntable
12: Drive mechanism
14: Collected blood amount measurement mechanism
15: Hand
16: Control mechanism
18: Input and output device
17: Pressure adjustment mechanism
111: Puncture device holder
112: Complete blood count blood collection tube holder
113: Biochemistry/immunology blood collection tube holder
114: Gauze holder
115: Adhesive bandage holder
120: Rotation direction drive mechanism
121: Vertical direction drive mechanism
130: Finger compression mechanism
131: Finger rest
132: Compression component
133: Tapered compression component
134: Adjustable compression component
153: Finger
171: Compression cuff pressure sensor
172: Compression cuff valve
173: Compression cuff pump
191: Finger blood vessel image capture camera
192: Finger blood vessel image capture light source
1111: Puncture device
1121: Complete blood count blood collection tube
1131: Biochemistry/immunology blood collection tube
1141: Gauze
1151: Adhesive bandage
1301: Base section compression cuff
1302: Middle section compression cuff
1303: Distal section compression cuff
1321: Finger dorsal surface fixing unit
1322: Finger pad compression unit
1331: Tapered finger dorsal surface fixing unit
1342: Stretchable unit

## Claims

1. A blood collection device that collects blood from a puncture mark on a fingertip by repeatedly compressing a finger in order of a base section and/or a middle section and a distal section of the finger, the blood collection device comprising a compression mechanism that independently compresses the base section and/or the middle section and the distal section of the finger, wherein
a period of time for compressing the distal section is longer than a period of time for compressing the middle section and/or a period of time for compressing the base section.

2. The blood collection device according to claim 1, further comprising:
an image capture mechanism that creates an image of the fingertip; and an analysis mechanism that analyzes the image created by the image capture mechanism and determines a bleeding condition, wherein
the period of time for compressing the distal section by the compression mechanism is increased or decreased based on a result of the analysis by the analysis mechanism.

3. The blood collection device according to claim 1, further comprising:
an image capture mechanism that creates an image of a blood vessel of the fingertip; and an analysis mechanism that analyzes the image created by the image capture mechanism and determines a condition of the blood vessel of the fingertip, wherein
the period of time for compressing the distal section by the compression mechanism is increased or decreased based on a result of the analysis by the analysis mechanism.

4. The blood collection device according to claim 2, further comprising:
a puncture needle that punctures a puncture target site on the fingertip; a blood collection container for collecting blood from a puncture mark of the puncture target site; gauze to be in contact with the puncture mark; a sticker for sealing the puncture mark; a drive mechanism that changes relative positions of a puncture device, the blood collection container, the gauze, and the sticker to the puncture target site on a horizontal plane and changes relative positions of the puncture device, the blood collection container, the gauze, and the sticker to the puncture target site in a vertical direction; and a compression mechanism for compressing a portion of the puncture target site.

5. The blood collection device according to claim 3, further comprising:
a puncture needle that punctures a puncture target site on the fingertip; a blood collection container for collecting blood from a puncture mark of the puncture target site; gauze to be in contact with the puncture mark; a sticker for sealing the puncture mark; a drive mechanism that changes relative positions of a puncture device, the blood collection container, the gauze, and the sticker to the puncture target site on a horizontal plane and changes relative positions of the puncture device, the blood collection container, the gauze, and the sticker to the puncture target site in a vertical direction; and a compression mechanism for compressing a portion of the puncture target site.

6. The blood collection device according to claim 4, wherein
the compression mechanism for the distal section includes a fixing unit that fixes the finger by being brought into contact with a dorsal surface and a side surface of the finger, and
a finger pad compression unit that is disposed on a palmar side of the finger and compresses a pad of the finger, and
the compression mechanism for the distal section changes a relative distance between the fixing unit and the finger pad compression unit.

7. The blood collection device according to claim 5, wherein
the compression mechanism for the distal section includes a fixing unit that fixes the finger by being brought into contact with a dorsal surface and a side surface of the finger, and
a finger pad compression unit that is disposed on a palmer side of the finger and compresses a pad of the finger, and
the compression mechanism for the distal section changes a relative distance between the fixing unit and the finger pad compression unit.

8. The blood collection device according to claim 6, wherein
the fixing unit has a shape in which a distance between surfaces that come into contact with both side surfaces of the finger becomes shorter toward a dorsal side of the finger.

9. The blood collection device according to claim 7, wherein
the fixing unit has a shape in which a distance between surfaces that come into contact with both side surfaces of the finger becomes shorter toward a dorsal side of the finger.

10. The blood collection device according to claim 8, wherein
an adjustable finger dorsal surface fixing unit that comes into contact with both side surfaces of the finger has a structure in which a distance between surfaces that come into contact with both side surfaces of the finger is adjustable.

11. The blood collection device according to claim 9, wherein
an adjustable finger dorsal surface fixing unit that comes into contact with both side surfaces of the finger has a structure in which a distance between surfaces that come into contact with both side surfaces of the finger is adjustable.

12. A blood collection method of collecting blood from a puncture mark on a fingertip by causing a compression mechanism configured to independently compress a base section and/or a middle section and a distal section of a finger to repeatedly compress the finger in order of the base section and/or the middle section and the distal section, wherein
a period of time for compressing the distal section is longer than a period of time for compressing the middle section and/or a period of time for compressing the base section.

13. The blood collection method according to claim 12, wherein
an image capture mechanism that creates an image of the fingertip, and an analysis mechanism that analyzes the image created by the image capture mechanism and determines a bleeding condition are further provided, the blood collection method further comprising:
causing the image capture mechanism to create an image of the fingertip; and
causing the analysis mechanism to analyze the image created by the image capture mechanism, determine a bleeding condition, and increase or decrease the period of time for compressing the distal section based on a result of the determination.

14. The blood collection method according to claim 12, wherein
an image capture mechanism that creates an image of the fingertip, and an analysis mechanism that analyzes the image created by the image capture mechanism and determines a bleeding condition are further provided, the blood collection method further comprising:
causing the image capture mechanism to create an image of a blood vessel of the fingertip; and
causing the analysis mechanism to analyze the image created by the image capture mechanism, determine a condition of the blood vessel of the fingertip, and increase or decrease the period of time for compressing the distal section based on a result of the determination.
